# EUROPEAN PATENT APPLICATION

(11) **EP 4 520 779 A1**
(43) Date of publication of application: **12.03.2025**
(21) Application number: 23799499.1
(22) Date of filing: 01.05.2023
(51) Int. Cl.: C08J 3/205, C08J 3/02, C08K 5/32, C08K 5/357, C08L 1/02, C08L 29/04, C08L 89/00

(54) **METHOD FOR DISSOLVING SPARINGLY WATER-SOLUBLE PROTEIN, PROTEIN SOLUTION OBTAINED BY METHOD FOR DISSOLVING SPARINGLY WATER-SOLUBLE PROTEIN, AND MOLDED BODY OF PROTEIN SOLUTION OBTAINED BY METHOD FOR DISSOLVING SPARINGLY WATER-SOLUBLE PROTEIN**

(30) Priority: 02.05.2022 JP 2022076053
(71) Applicant: Pharma Foods International Co., Ltd., Kyoto 615-8245 (JP)
(72) Inventor: SHINODA, Katsumi, Kyoto-shi, Kyoto 615-8245 (JP); KOGA, Keita, Kyoto-shi, Kyoto 615-8245 (JP); KIM, Mujo, Kyoto-shi, Kyoto 615-8245 (JP)
(74) Representative: Uexküll & Stolberg
(86) International application number: PCT/JP2023/017082
(87) International publication number: WO 2023/214570

(57) **Abstract**

The present invention provides a method for dissolving a water-insoluble protein and a method for producing a polymer molded body containing a water-insoluble protein.

The present invention provides a method for dissolving a water-insoluble protein, the method including a step of mixing a water-insoluble protein with a water-soluble tertiary amine oxide. Further, in the present invention, a molded body can be produced from a protein solution obtained by such a method.

## Description

### TECHNICAL FIELD

The present invention relates to a method for dissolving a water-insoluble protein and a protein solution obtained by the method, and particularly to a method for dissolving a water-insoluble protein in a water-soluble tertiary amine oxide, a protein solution obtained by the method, and a molded body of the solution.

### BACKGROUND ART

In recent years, SDGs have attracted attention, and efforts have been made to effectively use resources that have been discarded to aim for a sustainable society and to sustain a rich environment without destroying the earth. As a part of the efforts, it is desired to dissolve a water-insoluble protein that has been difficult to reuse and discarded, and to use the protein as a protein solution.

### SUMMARY OF THE INVENTION

### SOLUTIONS TO THE PROBLEMS

The present inventors have found a method for dissolving a water-insoluble protein for recycling in addition to effectively utilizing discarded resources. Further, the present inventors have found a method for making a water-insoluble protein into a solution without reducing molecular weight of the protein by hydrolysis in order to express a novel function. Therefore, the present invention provides a method for dissolving a water-insoluble protein by using a water-soluble tertiary amine oxide.

Therefore, according to a main aspect of the present invention, an invention below is provided.

### (Item 1)

A method for dissolving a water-insoluble protein, the method including:
a step of mixing a water-insoluble protein with a water-soluble tertiary amine oxide.

### (Item 2)

The method according to the above item, in which the step of mixing includes mixing using high shear force and/or vibration.

### (Item 3)

The method according to any one of the above items, in which the water-insoluble protein is dissolved without being hydrolyzed.

### (Item 4)

The method according to any one of the above items, in which the water-soluble tertiary amine oxide includes N-methylmorpholine N-oxide (nMMO).

### (Item 5)

The method according to any one of the above items, in which the nMMO is at least about 70 w/w% nMMO.

### (Item 6)

The method according to any one of the above items, in which the water-insoluble protein includes an eggshell membrane, a chicken feather, wool, and a scale.

### (Item 7)

A protein solution obtained by dissolving a water-insoluble protein in a water-soluble tertiary amine oxide.

### (Item 8)

The protein solution according to any one of the above items, in which the water-insoluble protein is dissolved without being hydrolyzed.

### (Item 9)

The protein solution according to any one of the above items, in which the water-soluble tertiary amine oxide includes an N-methylmorpholine N-oxide.

### (Item 10)

The protein solution according to any one of the above items, in which the nMMO is at least about 70 w/w% nMMO.

### (Item 11)

The protein solution according to any one of the above items, in which the water-insoluble protein includes an eggshell membrane, a chicken feather, wool, and a scale.

### (Item 12)

A method for producing a polymer molded body containing a water-insoluble protein, the method including:
(a) a step of mixing a water-insoluble protein with a water-soluble tertiary amine oxide;
(b) a step of mixing a hydroxyl group-containing polymer with a water-soluble tertiary amine oxide;
(c) a step of mixing a protein solution obtained by the step (a) with polymer solution obtained by the step (b); and
(d) a step of precipitating a polymer molded body from a solution obtained by the step (c).

### (Item 13)

The method according to any one of the above items, in which the hydroxyl group-containing polymer includes a modified hydroxyl group-containing polymer having a functional group attached to the hydroxyl group-containing polymer.

### (Item 14)

The method according to any one of the above items, in which the hydroxyl group-containing polymer includes saponified product of polyvinyl acetate, polyvinyl alcohol, and cellulose.

### (Item 15)

The method according to any one of the above items, in which the water-soluble tertiary amine oxide includes N-methylmorpholine N-oxide (nMMO).

### (Item 16)

The method according to any one of the above items, in which the nMMO is at least about 70 w/w% nMMO.

### (Item 17)

The method according to any one of the above items, in which the water-insoluble protein includes an eggshell membrane, a chicken feather, wool, and a scale.

### (Item 18)

A polymer molded body containing a water-insoluble protein, the molded body obtained by a method including:
(a) a step of mixing a water-insoluble protein with a water-soluble tertiary amine oxide;
(b) a step of mixing a hydroxyl group-containing polymer with a water-soluble tertiary amine oxide;
(c) a step of mixing a protein solution obtained by the step (a) with a polymer solution obtained by the step (b); and
(d) a step of precipitating a polymer molded body from a solution obtained by the step (c).

In the present disclosure, it is intended that one or more of the features described above may be provided in further combination in addition to the disclosed combination. Note that further embodiments and advantages of the present disclosure will be recognized by those skilled in the art upon reading and understanding detailed description below as appropriate.

Note that a feature and a remarkable action and effect of the present disclosure other than those described above will be clear to those skilled in the art with reference to a section of an embodiment and drawings of the invention.

### ADVANTAGES OF THE INVENTION

The present invention can produce a protein solution which is excellent in effective use of resources of a water-insoluble protein such as an eggshell membrane, a chicken feather, wool, and a scale which are often discarded, and by which a molded body imparted with new functionality is easily obtained.

Since the protein solution and the molded body of the protein solution of the present invention can impart new functionality in addition to original properties, functionalities such as moisture absorption and release properties, deodorizing properties, and antibacterial properties can be provided.

Furthermore, since a shape and area of the molded body of the present invention are not limited, it is possible to eliminate a disadvantage of a natural protein that a molded body is limited to a shape corresponding to the original size of the protein.

### BRIEF DESCRIPTION OF THE DRAWINGS

Fig. 1 is a micrograph of a water-insoluble protein regenerated by using a protein solution produced in Example 3.
Fig. 2 is a micrograph of a polymer molded body prepared by using the protein solution produced in Example 7.
Fig. 3 is a micrograph of a polymer molded body prepared by using the protein solution produced in Example 8.

### MODE FOR CARRYING OUT THE INVENTION

Hereinafter, the present disclosure will be described while showing a best mode. It is to be understood that throughout the description, expression of singular forms also includes the concept of plural forms, unless otherwise stated. Therefore, it is to be understood that an article of a singular form (for example, in English, "a", "an", "the", and the like) also includes the concept of a plural form unless otherwise stated. Further, it is to be understood that terms used in the present description are used in the sense commonly used in the art unless otherwise noted. Therefore, unless defined otherwise, all technical and scientific terms used in the present description have the same meaning as commonly understood by a person skilled in the art to which the present disclosure belongs. In case of conflict, the present description (including definitions) will control.

Hereinafter, definitions and/or basic technical content of terms particularly used in the present description will be described as appropriate.

As used in the present description, "about" means ±10% of a subsequent numerical value.

In the present description, "water-insoluble protein" refers to a protein that is not transparently dissolved even when a 0.1% aqueous solution is heated at 80°C for three hours. As a method for determining whether or not a protein is a water-insoluble protein, whether or not the above condition is satisfied can be checked using a conventional method. Examples of the water-insoluble protein include an eggshell membrane, a chicken feather, wool, and a scale.

In the present description, "protein solution" obtained by dissolving a water-insoluble protein is interpreted in a broad sense, and refers to a solution in which a water-insoluble protein is mainly dissolved.

In the present description, "water-soluble tertiary amine oxide" is interpreted in a broad sense, and refers to a water-soluble amine oxide or amine-N-oxide. This refers to an oxide of a nitrogen-containing heterocyclic compound such as a tertiary amine or pyridine. Examples of the water-soluble tertiary amine oxide include N-methylmorpholine N-oxide (nMMO), and nMMO is a kind of organic compound and is used as an oxidant in organic synthesis. This nMMO corresponds to an amine oxide of N-methylmorpholine (NMM) in which a methyl group is substituted on nitrogen of morpholine.

In the present description, "polymer molded body" is interpreted in a broad sense, and refers to a molded body containing a polymer as a main component.

In the present description, "hydroxyl group-containing polymer" is interpreted in a broad sense, and refers to a polymer compound containing a hydroxyl group. Examples of the hydroxyl group-containing polymer include polysaccharides such as starch, glycogen, cellulose, chitin, agarose, hyaluronic acid, chondroitin sulfate, pectin, and carrageenan, polyvinyl alcohol (PVA), and synthetic polymers such as phenol resin and polyvinyl acetate. Further, examples of the hydroxyl group-containing polymer can also include a partially saponified product of polyvinyl acetate and regenerated cellulose.

In the present description, mixing using "high shear force" refers to mixing liquid or liquid and solid while shearing with a stirring blade that rotates at a high speed. Examples of a stirrer capable of obtaining high shear force include a high-speed shear mixer such as a homogenizer, a turbo type stirrer, or a biomixer, a highpressure homogenizer, an ultrasonic homogenizer, or an in-line mixer (static mixer and injection molding mixer (one axis, two axes)).

In the present description, mixing using "vibration" is interpreted in a broad sense, and refers to stirring liquid or solid and a solvent while vibrating in a container containing the liquid or the solid to be mixed. Mixing using vibration can be achieved, for example, by stirring with a stirrer, stirring by ultrasonic vibration, stirring by vibration at a molecular level, or the like.

In the present description, a "dissolved" state includes a state in which an appearance of a solution is transparent, translucent, or emulsified.

### (Preferred embodiment)

A preferred embodiment of the present disclosure will be described below. The embodiment provided below is provided for a better understanding of the present disclosure, and the scope of the present disclosure should not be limited to description below. Therefore, it is clear that those skilled in the art can appropriately make modification within the scope of the present disclosure in view of the description in the present description. Further, the embodiments below of the present disclosure can be used alone or in combination.

According to an aspect of the present invention, there is provided a method for dissolving a water-insoluble protein, the method including a step of mixing a water-insoluble protein with a water-soluble tertiary amine oxide.

### [Water-insoluble protein]

In one embodiment, the method for the present invention can dissolve a water-insoluble protein by mixing the water-insoluble protein with a water-soluble tertiary amine oxide.

In one embodiment, a water-insoluble protein refers to a protein that does not dissolve transparently even when a 0.1% aqueous solution of the protein is heated at 80°C for three hours, and examples of the water-insoluble protein include an eggshell membrane, a chicken feather, wool, and a scale.

In one embodiment, an eggshell membrane can be used as a water-insoluble protein. The origin of the eggshell membrane as a water-insoluble protein dissolved by the method for the present invention is not particularly limited. For example, as the eggshell membrane, an eggshell membrane of poultry such as a chicken, a quail, a turkey, a duck, a goose, an ostrich, a guinea fowl, a guinea fowl, a phoenix chicken, a bantam, a pigeon, a mute swan, an emu, or a pheasant can be used. In one embodiment of the present invention, chickens are preferable in that they are easily available, are produced abundantly as egg-laying species, and have large eggs, and a method for breeding many chickens is established.

Usually, an eggshell adheres to an eggshell membrane, and if the eggshell is hydrolyzed as it is, the eggshell remains insoluble and exists as a foreign substance when a molded body is obtained, which is not preferable. In order to remove the eggshell, the eggshell membrane containing the eggshell is preferably immersed in an aqueous solution of hydrochloric acid, phosphoric acid, or the like, the eggshell is dissolved in water, filtered, and washed with water, so that the eggshell is removed in advance.

### [Pulverization of water-insoluble protein]

In one embodiment, a water-insoluble protein can be pulverized to facilitate dissolution of the water-insoluble protein. In one embodiment, size of a diameter in a case where a water-insoluble protein is pulverized is not particularly limited, but for example, the diameter of the water-insoluble protein in a case of being pulverized can be about 0.1 µm or more, about 0.2 µm or more, about 0.3 µm or more, about 0.4 µm or more, about 0.5 µm or more, about 0.6 µm or more, about 0.7 µm or more, about 0.8 µm or more, about 0.9 µm or more, about 1.0 µm or more, about 1.5 µm or more, about 2.0 µm or more, about 3.0 µm or more, about 4.0 µm or more, or about 5.0 µm or more, and the diameter can be about 5 mm or less, about 4 mm or less, about 3 mm or less, about 2 mm or less, or about 1 mm or less. In one embodiment, in a case of being pulverized, a water-insoluble protein preferably has a diameter of about 0.1 µm or more and about 5 mm or less, and more preferably about 1 µm or more and about 1 mm or less. Within this range, a water-insoluble protein can be uniformly dissolved in a case of obtaining a solution by using the method for the present invention.

### [Dissolution of water-insoluble protein]

In one embodiment, the method for the present invention can dissolve a water-insoluble protein by mixing the water-insoluble protein with a water-soluble tertiary amine oxide, for example, by applying high shear force and/or applying vibration to a mixed solution of the water-insoluble protein and the water-soluble tertiary amine oxide.

In one embodiment, examples of a solvent that dissolves a water-insoluble protein include a water-soluble tertiary amine oxide, and as the water-soluble tertiary amine oxide, for example, N-methylmorpholine N-oxide (nMMO) can be used. This nMMO is preferable from the viewpoint of solubility of a water-insoluble protein, and is also preferable from the viewpoint that nMMO can be reused after a molded body is regenerated from a water-insoluble protein converted into a solution as described later. In one embodiment, in a case where a water-insoluble protein is dissolved in N-methylmorpholine N-oxide (nMMO), a solution of at least about 70 w/w%, at least about 75 w/w%, at least about 80 w/w%, at least about 85 w/w%, at least about 90 w/w%, at least about 95 w/w%, at least about 97 w/w%, at least about 98 w/w%, and at least about 99 w/w% can be used as an nMMO solution. In one embodiment, an nMMO solution of about 80 w/w% or more can be preferably used.

The nMMO is a kind of organic compound, and is colorless solid used as an oxidant in organic synthesis. This corresponds to an amine oxide of N-methylmorpholine (NMM) in which a methyl group is substituted on nitrogen of morpholine. It is also used as a sacrificial reagent in a catalytic oxidation reaction with osmium tetroxide, or in Sharpless oxidation, TPAP oxidation, or the like. The nMMO serves as a reoxidant that reoxidizes and reactivates a catalyst in a state of having less activity after oxidizing a substrate. The nMMO is usually commercially available as a 50% aqueous solution. One that is anhydrous is also available, but an aqueous solution that is dehydrated and dried is also sometimes used. Since an aqueous solution dissolves cellulose, it is also used as a solvent for lyocell production.

In one embodiment, concentration of a water-insoluble protein in a case of dissolving the water-insoluble protein is not particularly limited, but is preferably about 0.1% to about 20%, and can be, for example, about 0.1%, about 0.2%, about 0.5%, about 0.8%, about 1.0%, about 1.5%, about 2.0%, about 2.5%, about 3.0%, about 3.5%, about 4.0%, about 4.5%, about 5.0%, about 6.0%, about 7.0%, about 8.0%, about 9.0%, about 10%, about 11%, about 12%, about 13%, about 14%, about 15%, about 16%, about 17%, about 18%, about 19%, or about 20%.

In one embodiment, temperature of a solution in a case of dissolving a water-insoluble protein is not particularly limited, but is preferably about 40°C or higher, and more preferably about 45°C or higher, 50°C or higher, about 55°C or higher, 65°C or higher, about 70°C or higher, 75°C or higher, about 80°C or higher, or about 90°C or higher.

In one embodiment, in a case where a water-insoluble protein is dissolved, a solution obtained by mixing the water-insoluble protein and a water-soluble tertiary amine oxide can be stirred according to a conventional method, and for example, the solution can be stirred using a conventionally known stirrer. In one embodiment, a solution obtained by mixing a water-insoluble protein and a water-soluble tertiary amine oxide can be stirred using a stirrer having high shear force such as a high viscosity stirrer or a homomixer, or an ultrasonic stirrer or the like can also be used.

In one embodiment, in the method for the present invention, a water-insoluble protein can be dissolved without being hydrolyzed. In this case, the water-insoluble protein is preferably pulverized to a diameter of the size as described above and then mixed with a water-soluble tertiary amine oxide and dissolved.

In a conventional technique, in order to convert a water-insoluble protein into a solution, the water-insoluble protein is broken down into smaller molecules by hydrolysis and dissolved. A protein is hydrolyzed by acid (hydrochloric acid, sulfuric acid, or the like), alkali (sodium hydroxide, potassium hydroxide, or the like), or protease (enzyme of microbial origin, enzyme of plant origin, enzyme of animal origin), for example, a solubilized protein having a relatively large molecular weight in a case of hydrolyzation at high temperature in the presence of alkali has been proposed (see, for example, Patent No. 6288686). Further, a protein hydrolysate having high cystine content by allowing protease having optimum pH to act on an alkaline region has also been proposed (see, for example, JP-A-2018-177713).

Further, a technique of a fiber assembly in which a protein is dissolved in thiopropionic acid or the like and spun by an electrospinning method has been proposed (see, for example, JP-A-2009-89859 and Patent No. 5166953), and a method for processing a solubilized protein obtained by hydrolyzing a protein in the presence of a reducing agent into fiber fabric has also been proposed (see, for example, Patent No. 4387806 and JP-A-2004-84154).

However, in the hydrolysis in the presence of alkali described in Patent No. 6288686, a step of neutralizing with an acidic substance is required in order to stop decomposition reaction, and there is a problem that content of cystine contained in a protein decreases in the neutralization step. In addition, it is difficult to obtain a solubilized product of a protein having a large molecular weight by hydrolysis with an enzyme described in JP-A-2018-177713, and protease itself as a catalyst is expensive, so that production cost increases.

Further, in the method for hydrolysis using acid, there have been problems that a device is easily corroded, a protein is excessively decomposed, molecular weight is excessively reduced by deviating from target molecular weight distribution, a protein is colored in brown, and a byproduct is easily generated in addition to target hydrolysate.

Furthermore, in a fiber manufacturing method described in JP-A-2009-89859 and Patent No. 5166953, a protein is dissolved in acid and then spun using a special device, and a fiber assembly does not have a natural protein structure. Further, in a film and a sheet described in Patent **No.** 4387806 and JP-A-2004-84154, a hydrolyzed solubilized protein is used, and the use is limited to a wound covering material such as an adhesive bandage.

Further, Patent No. 4070579 also proposes, as a method for utilizing a water-insoluble protein, functional rayon obtained by dissolving a water-insoluble protein such as animal hair at pH8 to pH12, mixing a crosslinked protein crosslinked with a water-soluble crosslinking agent in an aqueous alkali solution with viscose, and spinning the mixture, in order to introduce a functional component into viscose rayon. However, also in this method, a protein obtained by alkali hydrolysis is crosslinked with a crosslinking agent, and is different from an original protein, and in this respect, the technique is not different from other conventional techniques.

Further, in Patent No. 5071613, in order to attach a water-insoluble active ingredient (for example, titanium dioxide, zinc oxide, or the like) to a target surface of a tooth, it is proposed to dissolve the water-insoluble active ingredient in an aqueous solution containing an electrolyte by using an amphoteric polymer compound having an anionic dissociation group and a cationic dissociation group in one molecule, and a combination of an anionic polymer compound or the like and a cationic polymer compound or the like. However, a water-insoluble protein is not dissolved in this technique.

Therefore, in one embodiment of the present invention, in the method for the present invention, a water-insoluble protein can be dissolved without being hydrolyzed, so that it can have an original structure and characteristic of a natural protein. Further, by not hydrolyzing a water-insoluble protein, molding such as fiberization can be performed with a high molecular weight, so that strength of a molded body can be made high, and content of a protein can also be made high. Furthermore, for example, in a case of using an eggshell membrane as a water-insoluble protein, since a fibrous form remains as it is, structural characteristics such as moisture retention, deodorization, and increase in cell proliferation can be expected.

### [Protein solution of water-insoluble protein]

In one aspect of the present invention, there is provided a protein solution obtained by dissolving a water-insoluble protein in a water-soluble tertiary amine oxide. The protein solution of the present invention is obtained by dissolving a water-insoluble protein, and since the water-insoluble protein is originally less soluble in water, the water-insoluble protein is not soluble in water even when gel permeation chromatography is used, and it is not possible or practical to measure molecular weight. Further, it is also difficult to decompose a protein solution obtained by dissolving a water-insoluble protein in a water-soluble tertiary amine oxide to identify its amino acid sequence.

In one embodiment of the present invention, the protein solution of a water-insoluble protein of the present invention can be a protein solution obtained without generating a protein derivative such as hydrolysis of the water-insoluble protein. In one embodiment, the protein solution of a water-insoluble protein of the present invention can be obtained by dissolving the water-insoluble protein in an aqueous solution of a water-soluble tertiary amine oxide and gelling the solution.

As the water-soluble tertiary amine oxide, concentration of the water-soluble tertiary amine oxide, and the water-insoluble protein, those described in other places in the present description can be used.

### [Regeneration of solubilized water-insoluble protein]

In one aspect of the present invention, there is provided a method for producing a polymer molded body containing a water-insoluble protein, the method including (a) a step of mixing a water-insoluble protein with a water-soluble tertiary amine oxide, (b) a step of mixing a hydroxyl group-containing polymer with a water-soluble tertiary amine oxide, (c) a step of mixing a protein solution obtained by the step (a) with a polymer solution obtained by the step (b), and (d) a step of precipitating a polymer molded body from a solution obtained by the step (c).

In an embodiment of the present invention, by extruding a solubilized water-insoluble protein into water or an aqueous solution of a water-soluble tertiary amine oxide of less than about 60 w/w%, the water-insoluble protein can be precipitated and regenerated. For example, a water-insoluble protein dissolved in nMMO is extruded into an aqueous nMMO solution of less than about 60 w/w% to obtain a short fiber shape (cotton shape), or a water-insoluble protein dissolved in nMMO is applied in a thin film shape with a glass plate or the like and immersed in an aqueous nMMO solution of less than about 60 w/w% to be precipitated in a film shape, so that the water-insoluble protein can be regenerated. In one embodiment, a shape to be regenerated may be a cotton shape or a film shape, and a final product can be obtained by further washing with water and drying after regeneration.

Therefore, in one aspect of the present invention, there is provided a molded body, which is a polymer molded body containing a water-insoluble protein, the molded body obtained by a method including (a) a step of mixing a water-insoluble protein with a water-soluble tertiary amine oxide, (b) a step of mixing a hydroxyl group-containing polymer with a water-soluble tertiary amine oxide, (c) a step of mixing a protein solution obtained by the step (a) with a polymer solution obtained by the step (b), and (d) a step of precipitating a polymer molded body from a solution obtained by the step (c). As described above, the protein solution of the present invention is obtained by dissolving a water-insoluble protein, and since the water-insoluble protein is originally less soluble in water, the water-insoluble protein is not soluble in water even when gel permeation chromatography is used, and it is not possible or practical to measure molecular weight. Further, it is also difficult to decompose a protein solution obtained by dissolving a water-insoluble protein in a water-soluble tertiary amine oxide to identify its amino acid sequence. Therefore, it is also difficult to express a polymer molded body obtained from such a protein solution of the present invention by a structure and a characteristic.

In one embodiment, the polymer molded body is not particularly limited as long as it is a molded body obtained from a hydroxyl group-containing polymer to be a material of the method for the present invention, and examples of the polymer molded body include a fiber, nonwoven fabric, and a film.

Examples of characteristics of a molded body obtained by the method for the present invention include moisture absorption and release properties, deodorizing properties, antibacterial properties, skin protective properties, cell proliferation properties, biocompatibility, and metal adsorption properties.

In one embodiment, in the method for producing a polymer molded body of the present invention, in order to mix a water-insoluble protein and a hydroxyl group-containing polymer, it is preferable to mix and dissolve each of a water-insoluble protein and a hydroxyl group-containing polymer in a water-soluble tertiary amine oxide.

In one embodiment, in the method for producing a polymer molded body of the present invention, a water-soluble tertiary amine oxide serving as a solvent for a water-insoluble protein and a water-soluble tertiary amine oxide serving as a solvent for a hydroxyl group-containing polymer may be the same or different. In one embodiment, as a water-soluble tertiary amine oxide, for example, N-methylmorpholine N-oxide (nMMO) can be used. In order to reuse a water-soluble tertiary amine oxide, solvents for dissolving a water-insoluble protein and a hydroxyl group-containing polymer are preferably the same.

In one embodiment, an aqueous solution of a water-soluble tertiary amine oxide used for precipitation of a water-insoluble protein can be reused in the method for the present invention by concentration.

In one embodiment, a modified hydroxyl group-containing polymer in which a functional group is attached to a hydroxyl group-containing polymer can also be used as a hydroxyl group-containing polymer. By mixing an nMMO solution of a water-insoluble protein and an nMMO solution of a modified hydroxyl group-containing polymer and extruding the mixture into water or an aqueous solution of a water-soluble tertiary amine oxide of less than about 60 w/w%, a polymer molded body provided with functionality can be obtained.

In one embodiment, as a hydroxyl group-containing polymer or a modified hydroxyl group-containing polymer, a saponified product of polyvinyl acetate, polyvinyl alcohol, regenerated cellulose, and cellulose can be used, and regenerated cellulose can be preferably used. Further, a saponified product of polyvinyl acetate only needs to be saponified even at a part of polyvinyl acetate and contain a hydroxy (OH) group.

In one embodiment, a protein solution of a water-insoluble protein dissolved by the method for the present invention and a molded body of the protein solution have a function and a characteristic of the water-insoluble protein itself as a material, and can also have, for example, physiological activity such as fibroblast proliferation action, active enzyme removal action, and cell activation action in addition to moisturizing action, metal ion adsorption action, deodorizing action, antibacterial action, and the like. In one embodiment, a polymer material can also be selected so that functions and property of a water-insoluble protein can be easily exhibited.

In one embodiment of the present invention, the protein solution and the polymer molded body of the present invention are obtained from a water-insoluble protein such as an eggshell membrane derived from a natural component, and therefore have excellent adhesion to skin tissues of a human body.

In one embodiment of the present invention, since the protein solution and the polymer molded body of the present invention contain a component of a water-insoluble protein such as an eggshell membrane, the protein solution and the polymer molded body can have substantially the same property as that of a water-insoluble protein such as a natural eggshell membrane.

In the present description, "or" is used when "at least one or more" of matters listed in a sentence can be employed. The same applies to "or". In a case where, in the present description, "within a range" of "two values" is stated, the range includes the two values themselves.

References cited in the present description, such as scientific literature, a patent, a patent application, and the like, are hereby incorporated by reference in their entirety to the same extent as each is specifically described.

The present disclosure is described above with reference to a preferred embodiment for easy understanding. Hereinafter, the present disclosure will be described based on an example, but the above description and the example below are provided for the purpose of illustration only and not for the purpose of limiting the present disclosure. Accordingly, the scope of the present disclosure is not limited to the embodiment or example specifically described in the present description, but is limited only by the claims.

### EXAMPLES

### (Test example 1: dissolution of water-insoluble protein)

Wool, an eggshell membrane ground product, an eggshell membrane unground product, a scale, or a chicken feather was dissolved in 60 w/w%, 70 w/w%, or 85 w/w% N-methylmorpholine N-oxide (nMMO), or water according to Table 1 below. Protein concentration, stirring time, stirring and vibration time, and homogenizing time were as shown in Table 1.

**[Table 1]**

| | Example 1 | Example 2 | Example 3 | Example 4 | Example 5 | Example 6 | Example 7 | Example 8 | Example 9 | Example 10 | Example 11 | Comparative example 1 | Comparative example 2 | Comparative example 3 | Comparative example 4 |
|---|---|---|---|---|---|---|---|---|---|---|---|---|---|---|---|
| Wool | 5.0 | 5.0 | | | | | | | | 3.0 | | 5.0 | | | 3.0 |
| Eggshell membrane ground product | | | 6.5 | | | 6.5 | 3.0 | 5.0 | | | 3.0 | | 6.5 | | |
| Eggshell membrane unground product | | | | | | | | | 6.5 | | | | | | |
| Scale | | | | 7.0 | | | | | | | | | | 7.0 | |
| Chicken feather | | | | | 7.0 | | | | | | | | | | |
| Regenerated cellulose | | | | | | | 5.0 | 5.0 | | | | | | | |
| nMMO(85%) | 200 | 95 | 200 | 200 | 200 | 200 | 200 | 100 | 200 | | | | | | |
| nMMO(70%) | | | | | | | | | | 200 | 200 | | | | |
| nMMO(60%) | | | | | | | | | | | | | | | 200 |
| Water | | | | | | | | | | | | 200 | 200 | 200 | |
| Protein concentration | 2.4% | 5.0% | 3.1% | 3.4% | 3.4% | 3.1% | 1.4% | 4.5% | 3.1% | 1.5% | 1.5% | 3.1% | 3.4% | 3.5% | 1.5% |
| Stirring dissolution time | 20 hours | 20 hours | | | 20 hours | | 20 hours | | 40 hours | 20 hours | 20 hours | | | | 20 hours |
| Stirring and vibration dissolution time | | | 20 hours | | | | | | | | | 20 hours | | | |
| Homogenizing time | | | | 1 hour | | 30 minutes | | 1 hour | | | | | 1 hour | | |
| Appearance | Transparent | Transparent | Transparent | Translucent | Transparent | Transparent | Transparent | Transparent | Transparent | Transparent | Transparent | Insoluble | | | |

For dissolution of wool, a ground eggshell membrane, an unground eggshell membrane, a scale, or a chicken feather, each was mixed with nMMO(60 w/w%, 70 w/w%, or 85 w/w%) or water and stirred under a condition in Table 1. Further, an eggshell membrane was pulverized with a normal grinder such as a pulverizer or a hammer mill to a size of several tens of microns to several hundreds of microns. Pulverization is preferably performed after an eggshell membrane is washed and dried.

As a result, as shown in Table 1, it could be confirmed that all of Example 1 to 10 became transparent gel or translucent, and each water-insoluble protein was dissolved. On the other hand, it was confirmed that none of Comparative Examples 1 to 4 was dissolved.

Subsequently, an eggshell membrane was dissolved using a solvent other than nMMO. A pulverized eggshell membrane was prepared, mixed with each solvent, and dissolved by stirring at 80°C for one hour.

The solvents used and the results are shown in Table 2.

**[Table 2]**

| | | | | | |
|---|---|---|---|---|---|
| | Alcohols | Methanol | | | |
| | | Ethanol | | | |
| | | Hexafluoro-2-propanol | | | |
| | Ethers | Methyl ether | | | |
| | | Ethyl ether | | | |
| | | Petroleum ether | | | |
| | Ketones | Acetone | | | |
| | Esters | Ethyl acetate | | | |
| | | DMSO | | | |
| | | | | | |
| Insoluble | | | | | |
| | Hydrocarbons | Chloroform | | | |
| | | Dichloromethane | | | |
| | | Hexane | | | |
| | | Toluene | | | |
| | Amine oxide | 50% nMMO aqueous solution | | | |
| | | 85% pyridine N-oxide aqueous solution | | | |
| | | 85% coconut oil alkyl dimethylamine oxide aqueous solution | | | |
| | Supercritical CO2 | | | | |
| | Water | | | | |
| Soluble | Acid solution | Performic acid: 30%H2O2(20°C) | | | |
| | | Thiopropionic acid | | | |
| | Reducing agent | NaBH4/NaOH | | | |
| | Amine oxide | 85% nMMO aqueous solution | | | |
| | Alkaline solution | 1N NaOH-80% ethanol | | | |

As shown in Table 2, it can be seen that an eggshell membrane is insoluble in many solvents and is dissolved when hydrolyzed by acid or alkali. Further, it was also dissolved when 85 w/w% nMMO was used.

### (Test example 2: regeneration of water-insoluble protein from a water-insoluble protein solution, and preparation of polymer molded body)

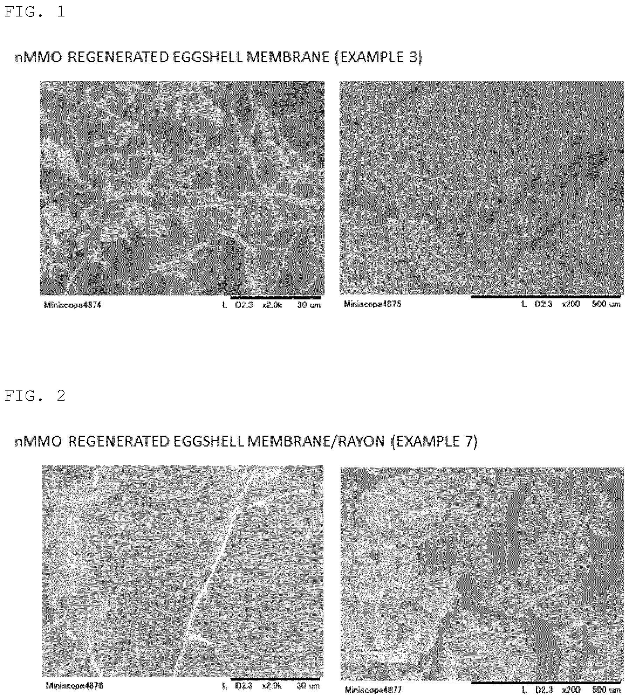

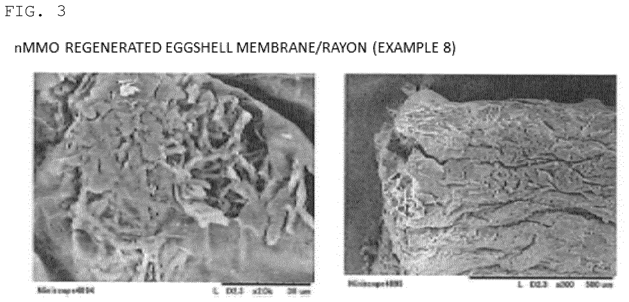

Protein solutions of Example 3, Example 7, and Example 8 described in Table 1 were used to regenerate a water-insoluble protein or prepare a polymer molded body. Each of eggshell membrane pulverized products was dissolved in an nMMO solution as in Examples 3, 7, or 8, then 4 g of the solution was taken on a glass plate, spread over as a thin film having a diameter of about 5 cm, insolubilized (formed into a film) in water, washed with water, and dried under reduced pressure at room temperature. For the protein solution of Example 3, a water-insoluble protein was regenerated as it was, and for the protein solutions of Examples 7 and 8, regenerated cellulose fibers (rayon) were mixed to form a film. Fig. 1 illustrates a micrograph of a film in a case where the protein solution of Example 3 was used, Fig. 2 illustrates a micrograph of a film in a case where the protein solution of Example 7 was used, and Fig. 3 illustrates a micrograph of a film in a case where the protein solution of Example 8 was used.

### (Note)

Although the present disclosure is illustrated using a preferred embodiment of the present disclosure as above, it is understood that the scope of the present disclosure should be interpreted only by the claims. It is understood that the patent, the patent application, and the document cited in the present description are to be incorporated by reference in the present description in the same manner as their content is specifically described in the present description. The present application claims priority to JP-A-2022-76053 filed on May 2, 2022 to the Japanese Patent Office, the entire content of which is incorporated in the present description by reference in the same manner as if its entirety constitutes content of the present application.

### INDUSTRIAL APPLICABILITY

A protein solution and a molded body of the protein solution obtained by the method for the present invention can be used in chemical product applications in the form of a fiber or a film, cosmetic applications for a face mask material or in the form of powder, medical applications as a wound covering material, and in the field of regenerative medicine as a cell culture scaffold material. Further, since biodegradability is excellent, use as an agricultural material can also be expected.

## Claims

1. A method for dissolving a water-insoluble protein, the method comprising:
a step of mixing a water-insoluble protein with a water-soluble tertiary amine oxide.

2. The method for claim 1, wherein
the step of mixing includes mixing using high shear force and/or vibration.

3. The method according to claim 1 or 2, wherein
the water-insoluble protein is dissolved without being hydrolyzed.

4. The method according to any one of claims 1 to 3, wherein
the water-soluble tertiary amine oxide includes N-methylmorpholine N-oxide (nMMO).

5. The method according to claim 4, wherein
the nMMO is at least about 70 w/w% nMMO.

6. The method according to any one of claims 1 to 5, wherein
the water-insoluble protein includes an eggshell membrane, a chicken feather, wool, and a scale.

7. A protein solution obtained by dissolving a water-insoluble protein in a water-soluble tertiary amine oxide.

8. The protein solution according to claim 7, wherein
the water-insoluble protein is dissolved without being hydrolyzed.

9. The protein solution according to claim 7 or 8, wherein
the water-soluble tertiary amine oxide includes an N-methylmorpholine N-oxide.

10. The protein solution according to claim 9, wherein
the nMMO is at least about 70 w/w% nMMO.

11. The protein solution according to any one of claims 7 to 10, wherein
the water-insoluble protein includes an eggshell membrane, a chicken feather, wool, and a scale.

12. A method for producing a polymer molded body containing a water-insoluble protein, the method comprising:
(a) a step of mixing a water-insoluble protein with a water-soluble tertiary amine oxide;
(b) a step of mixing a hydroxyl group-containing polymer with a water-soluble tertiary amine oxide,;
(c) a step of mixing a protein solution obtained by the step (a) with a polymer solution obtained by the step (b); and
(d) a step of precipitating a polymer molded body from a solution obtained by the step (c).

13. The method according to claim 12, wherein
the hydroxyl group-containing polymer includes a modified hydroxyl group-containing polymer having a functional group attached to the hydroxyl group-containing polymer.

14. The method according to claim 13, wherein
the hydroxyl group-containing polymer includes a saponified product of polyvinyl acetate, polyvinyl alcohol, and cellulose.

15. The method according to any one of claims 12 to 14, wherein
the water-soluble tertiary amine oxide includes N-methylmorpholine N-oxide (nMMO).

16. The method according to claim 15, wherein
the nMMO is at least about 70 w/w% nMMO.

17. The method according to any one of claims 12 to 16, wherein
the water-insoluble protein includes an eggshell membrane, a chicken feather, wool, and a scale.

18. A polymer molded body containing a water-insoluble protein, the molded body obtained by a method including:
(a) a step of mixing a water-insoluble protein with a water-soluble tertiary amine oxide;
(b) a step of mixing a hydroxyl group-containing polymer with a water-soluble tertiary amine oxide;
(c) a step of mixing a protein solution obtained by the step (a) with a polymer solution obtained by the step (b); and
(d) a step of precipitating a polymer molded body from a solution obtained by the step (c).
